(19) Europäisches Patentamt
European Patent Office
Office européen des brevets

(11) Veröffentlichungsnummer : **0 017 705**
**B1**

(12) # EUROPÄISCHE PATENTSCHRIFT

(45) Veröffentlichungstag der Patentschrift : **21.07.82**

(51) Int. Cl.³ : **C 07 C103/52**, C 07 C102/00, A 61 K 37/02

(21) Anmeldenummer : **80100423.5**

(22) Anmeldetag : **28.01.80**

(54) **Phenylalanyltryptophan-Derivate, sie enthaltende Arzneimittel, ihre Herstellung und Verwendung.**

(30) Priorität : **07.02.79 DE 2904512**

(43) Veröffentlichungstag der Anmeldung : **29.10.80 (Patentblatt 80/22)**

(45) Bekanntmachung des Hinweises auf die Patenterteilung : **21.07.82 Patentblatt 82/29**

(84) Benannte Vertragsstaaten : **AT BE CH DE FR GB IT LU NL SE**

(56) Entgegenhaltungen :
**FR - A - 2 375 867**
**FR - A - 2 376 128**

(73) Patentinhaber : **Troponwerke GmbH & Co. KG**
**Berliner Strasse 156**
**D-5000 Köln 80 (DE)**

(72) Erfinder : **Etschenberg, Eugen, Dr.**
**Virchowstrasse 20**
**D-5000 Koeln 41 (DE)**
Erfinder : **Jacobi, Haireddin**
**Finkenweg 2**
**D-5653 Leichlingen 1 (DE)**
Erfinder : **Opitz, Wolfgang, Dr.**
**Holzbachtalstrasse 60**
**D-5063 Overath-Steinenbrück (DE)**

(74) Vertreter : **Dill, Erwin, Dr. et al**
**c/o BAYER AG Zentralbereich Patente Marken und Lizenzen Bayerwerk**
**D-5090 Leverkusen 1 (DE)**

**EP 0 017 705 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Phenylalanyltryptophan-Derivate, sie enthaltende Arzneimittel, ihre Herstellung und Verwendung

Die vorliegende Erfindung betrifft neue Dehydropeptide, Verfahren zu ihrer Herstellung sowie ihre Verwendung als Arzneimittel, insbesondere als tumor- and/oder gewebsauflösende Arzneimittel.

Einige Dehydrooligopeptide und ihre tumor- bzw. gewebsauflösende Wirkung werden bereits in den DOS 2 659 114 und DOS 2 659 154 allgemein beschrieben ; jedoch sind die dort aufgeführten Verbindungen in ihrem Wert dadurch eingeschränkt, als sie bei der lokalen Applikation starke Schmerzen verursachen, die auch durch die gleichzeitige Gabe von Analgetika nur unvollständig unterdrückt werden können.

Aus FR-A 2 375 867 ist die Verbindung N-Benzoyldehydrophenylalanin-L-tryptophan bekannt. Vergleichende Untersuchungen zwischen der erfindungsgemäßen Verbindung des Beispiels 2 und dieser bekannten Verbindung zeigen beispielhaft, daß die erfindungsgemäßen Verbindungen eine nicht vorhersehbare vorteilhafte necrogene Wirkung zeigen, die sich quantitativ signifikant von der Wirkung der bekannten Verbindung unterscheiden.

Diese Erfindung betrifft Dehydropeptide der allgemeinen Formel I

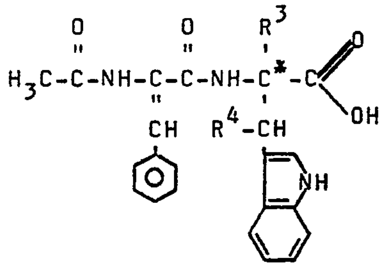

(I)

in welcher

$R^3$ und $R^4$ jeweils für ein Wasserstoffatom oder zusammen für eine zusätzliche Bindung zwischen den beiden C-Atomen stehen,

in ihrer racemischen und in ihrer optisch aktiven Form, sowie ihre physiologisch verträglichen Salze.

Die Herstellung der Verbindungen der allgemeinen Formel I erfolgt in an sich bekannter Weise, dadurch, daß man

a) für Verbindungen, in denen $R^3$ und $R^4$ jeweils Wasserstoff bedeuten,

Oxazole der Formel II

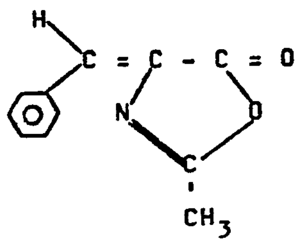

(II)

mit Tryptophanderivaten der Formel III

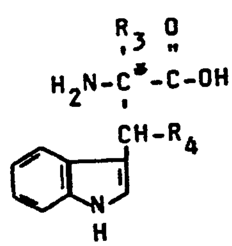

(III)

in welcher

$R^3$ und $R^4$ jeweils Wasserstoff bedeuten und

C* in der Racemat- oder in D- oder L-Form vorliegt,

in Gegenwart von organischen Lösungsmitteln, Wasser oder Gemischen aus beiden, bei Temperaturen zwischen 0 und 80 °C umsetzt oder

b) für Verbindungen der allgemeinen Formel I, in welcher

$R^3$ und $R^4$ für eine zusätzliche Bindung zwischen den beiden C-Atomen stehen,

gesättigte Oxazole der Formel IV

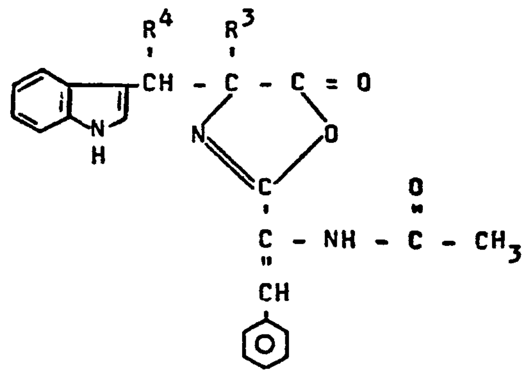

in welcher

$R^3$ und $R^4$ für jeweils ein Wasserstoffatom stehen,

in Gegenwart von inerten organischen Lösungsmitteln und von tertiären Aminen oxidiert und anschließend in üblicher Weise in Gegenwart von Wasser enthaltenden Verdünnungsmitteln bei Temperaturen zwischen 0 und 70 °C zu Verbindungen der allgemeinen Formel I verseift.

Die Herstellung der Verbindungen der Formel (I) gemäß Variante a) erfolgt vorzugsweise durch einfaches Verrühren der Reaktionskomponenten (II) und (III) in geeigneten Verdünnungsmitteln.

Als Verdünnungsmittel eignen sich besonders solche organischen Flüssigkeiten die polar aprotisch sind und mit Wasser vermischbar sind. Beispielhaft seien genannt Aceton, Methylethylketon, Diethylketon, niedere aliphatische Alkohole, cyclische Ether, insbesondere Dioxan und Tetrahydrofuran. Besonders bewährt haben sich Aceton und Tetrahydrofuran.

Verwendet man die freien Aminosäuren der Formel (III) als Kondensationspartner des Oxazolons der Formel (II), so setzt man bevorzugt auf 1 Mol Oxazolon 1 Mol Aminosäure und 1 Mol Natriumhydroxid ein. Die Reaktionstemperaturen können in gewissem Bereich variiert werden; im allgemeinen arbeitet man zwischen 0 und 80 °C, vorzugsweise bei 10 bis 30 °C.

Die Reaktionsdauer ist unterschiedlich und beträgt zwischen 30 Minuten und mehreren Tagen, vorzugsweise ein bis drei Stunden. Die Aufarbeitung erfolgt durch Filtrieren der Reaktionslösung und Ansäuern mit verdünnten Mineralsäuren. Besonders bevorzugt ist die Verwendung von 1 Mol Mineralsäure auf 1 Mol eingesetztes Natriumhydroxid. Die Isolierung der Reaktionsprodukte erfolgt üblicherweise durch Abdampfen des organischen Verdünnungsmittels im Vakuum, wobei das Reaktionsprodukt ausfällt, welches aus einem geeigneten Lösungsmittel umkristallisiert wird.

Die Ausgangsverbindungen (II) für die Herstellung der erfindungsgemäßen Verbindungen der allgemeinen Formel (I), die entsprechenden 2,4-disubstituierten 5(4H)-Oxazolone, sind literaturbekannt oder lassen sich nach bekannten Methoden herstellen (vgl. R.M. Herbst, D. Shemin in Org. Synth. Coll. Vol. II, 1 (1943).

Beispielshaft sei hier die Reaktion von Acetylglycin mit Benzaldehyd beschrieben. Die Reaktion erfolgt nach der Gleichung

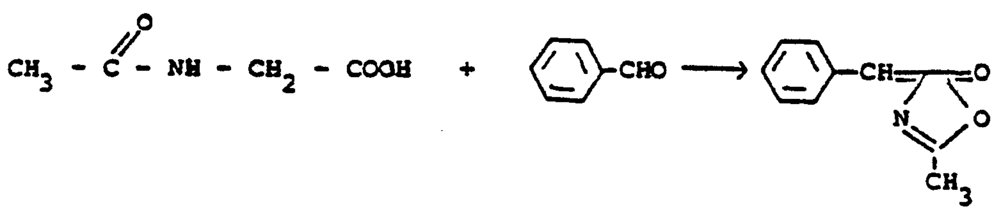

Die Reaktion erfolgt durch Mischen beider Komponenten in äquimolarem Verhältnis in Gegenwart von Kondensationsmitteln, vorzugsweise Acetanhydrid, welche zugleich als Lösungsmittel dienen, und einer basischen Komponente wie Natriumacetat. Nach mehrstündigem Stehen erfolgt die Aufarbeitung durch Verdünnen mit Wasser und Rekristallisation des ausgefallenen 4-Benzyliden-2-methyl-5(4H)-oxazolons aus Äthylacetat/Petroläther.

Die Herstellung der erfindungsgemäßen Verbindungen der allgemeinen Formel (I) in denen $R_3$ und $R_4$ gemeinsam für eine Bindung stehen, nach Variante b), erfolgt nach bekannten Methoden (vgl. S. Konno et al, Synthesis *1978*, 598) indem man Verbindungen der allgemeinen Formel (I) in welcher $R_3$ und $R_4$ jeweils für ein Wasserstoffatom und $R_5$ für eine Hydroxylgruppe stehen, hergestellt nach Variante a) durch Umsetzung mit einem Wasser entziehenden Mittel in einem inerten organischen Lösungsmittel zunächst zum gesättigten Oxazolon der Formel (IV), umsetzt und dieses anschließend mit Oxidationsmitteln in inerten organischen Lösungsmitteln und in Gegenwart von tertiären Basen zum entsprechenden ungesättigten Oxazolon der Formel (IV), worin $R_3$ und $R_4$ zusammen für eine zusätzliche Bindung zwischen den beiden C-Atomen stehen, umsetzt, welches anschließend nach bekannten Methoden zu den angestrebten Verbindungen der Formel (I) verseift wird.

Als Wasser entziehendes Mittel sei beispielhaft genannt Dicyclohexylcarbodiimid und als Oxidationsmittel sei beispielhaft genannt 2.3-Dichlor-5.6-dicyan-p-benzo-chinon. Als tertiäre Base sei beispielhaft genannt 2.4.6-Trimethylpyridin. Als Lösungsmittel seien beispielhaft genannt Tetrahydrofuran und 2.3-Dimethoxyethan.

Die Hydrolyse erfolgt durch Rühren bzw. Stehen mit Wasser in Gegenwart von Verdünnungsmitteln.

Als Verdünnungsmittel können sowohl polare aprotische als auch protische organische Verdünnungsmittel verwendet werden. Als polare aprotische Verdünnungsmittel seien beispielhaft genannt Ketone, wie z.B. Aceton, Methyläthylketon, Diäthylketon und cyclische Ether (Tetrahydrofuran, Dioxan). Besonders bevorzugt sind Aceton und Tetrahydrofuran.

Als polare protische Verdünnungsmittel seien beispielhaft genannt Wasser und niedere Alkohole, wie Methanol, Äthanol, Propanol oder Isopropanol.

Zweckmäßig wird die Reaktion in Gegenwart saurer oder basischer Katalysatoren durchgeführt.

Als saure Katalysatoren können starke Mineralsäuren, wie Salzsäure oder Schwefelsäure oder starke organische Säuren, wie Benzolsulfonsäure oder Toluolsulfonsäure eingesetzt werden.

Als basische Katalysatoren haben sich anorganische Basen, wie Natrium- oder Kaliumhydroxid sowie starke organische Basen wie Triäthylamin bewährt. Besonders bevorzugt sind Natriumhydroxid und Kaliumhydroxid.

Die Reaktionstemperaturen können in großem Bereich variiert werden. Im allgemeinen arbeitet man zwischen 0 und 70 °C, vorzugsweise zwischen 10 und 30 °C.

Als Beispiele für die erfindungsgemäßen Verbindungen seien genannt :

N-Acetyldehydrophenylalanyl-L-tryptophan
N-Acetyldehydrophenylalanyl-D-tryptophan
N-Acetyldehydrophenylalanyl-dehydrotryptophan.

Die erfindungsgemäßen Wirkstoffe besitzen eine dosisabhängige tumor- und gewebsauflösende Wirkung bei lokaler und systemischer Applikation. Unter lokal seien hier die folgenden Applikationsarten verstanden :

subkutan, intrakutan, intratumoral, peritumoral, in Form von Externa in Salben, Gelen, Lotiones, Cremes, Injektionslösungen und Injektionssuspensionen.

Unter systemisch sei verstanden : intravenös, intraperitoneal, rectal, intrapleural, oral, zum Spülen von körperhöhlen und Harnblase.

Die Nekrosen entstehen meist in unmittelbarem Bereich der Applikationsstellen, gelegentlich auch fern davon (lymphogen). Überraschenderweise is der nekrotische Bereich, wenn er nach außen aufbricht, frei von putridem Material, selbst über einen längeren Zeitraum, obwohl bei den Versuchstieren Futter, kot, Sägespäne und anderes mit der offenen Wunde in Berührung kamen.

Das Tumorgewebe kann jedoch auch einschmelzen bei völlig intakter äußerer Haut.

Das nekrotische Gewebe ist von dem umgebenden gesunden Gewebe scharf abgegrenzt ; es wirkt makro- und mikroskopisch wie ausgestanzt.

Das allgemeine Verhalten der Versuchstiere wird durch die Größe der Nekrose nicht beeinflußt. Es kommt zu keiner Vergiftung des Gesamtorganismus.

Die Lebenszeit von Tumorzellen (Lyphom EL4) wird durch die erfindungsgemäßen Verbindungen in vitro gegenüber Kontrollen signifikant verkürzt.

Die $DL_{50}$ der erfindungsgemäßen Verbindungen liegt im Akutversuch bei der Intravenösen Injektion an Ratte und Maus bei 400 mg/kg. Nach intraperitonealer Applikation bei der Maus liegt die $DL_{50}$ über 2 000 mg/kg und bei der Ratte über 1 000 mg/kg.

Bei therapeutischem Einsatz bewegen sich die Dosierungen in der Größenordnung von 1 bis 100 mg/kg Körpergewicht, vorzugsweise 2 bis 40 mg/kg.

Die erfindungsgemäßen Wirkstoffe können in bekannter Weise in die für ihre Wirkungsart erforderlichen Formulierungen übergeführt werden, wie Salben, Pasten, Cremes, lösliche Pulver, Puder, Emulsionen, Suspensionen und Injektionslösungen.

Beispielhaft für eine Injektionslösung werden die erfindungsgemäßen Wirkstoffe gegebenenfalls unter Zuhilfenahme von Lösungsvermittlern in verdünnten physiologisch verträglichen Basen gelöst und durch Zugabe von physiologisch verträglichen Säuren in eine injizierbare Form von pH 6,8 bis 8,0, insbesondere 7 bis 7,4 gebracht.

Als physiologisch verträgliche Basen seien beispielhaft genannt anorganische Hydroxide, Carbonate, Hydrogencarbonate, insbesondere die von Natrium und Kalium.

Als physiologisch verträgliche Säuren seien beispielhaft erwähnt organische Säuren, wie Citronensäure, Oxalsäure, Milchsäure, Benzoesäure, Salicylsäure, Essigsäure oder auch anorganische Säuren, wie z.B. verdünnte Salz- oder Schwefelsäure.

Den erfindungsgemäßen Wirkstoffen können in den Formulierungen geeignete Hilfsstoffe beigemischt werden. Als solche gelten inerte, nicht toxische und pharmazeutisch geeignete feste, halbfeste und flüssige Trägerstoffe, Emulgier- und Dispergiermittel. Hierbei soll die therapeutisch wirksame Verbindung jeweils in einer Konzentration von 1 bis 90 Gew.-%, vorzugsweise von 5 bis 50 Gew.-%, vorhanden sein.

Zur vorliegenden Erfindung gehören pharmazeutische Zubereitungen, die neben nichttoxischen, inerten pharmazeutisch geeigneten Trägerstoffen einen oder mehrere erfindungsgemäße Wirkstoffe

enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Suppositorien, Injektionslösungen, Suspensionen und Emulsionen, Pasten, Salben, Gele, Cremes, Lotiones, Puder und Sprays genannt.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Traganth, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, zur Behandlung von solchen Geweben im human- und veterinärmedizinischen Bereich, die den Ablauf der normalen biologischen Funktionen hindern und stören.

Solche Gewebe sind z.B. :

gut- und bösartige Tumoren solider und cystischer Natur, Papillome, Adenome, Cyst-Adenome ; Adenokarzinome einschließlich solcher vom cirrhoesen Typ ; Basalzellkarzinome ; Sarkome wie z.B. Fibrosarkom, Liposarkom, Myxosarkom, Rhabdomyosarkom, Chondrosarkom, Lymphosarkom, Reticulumzellsarkom, sowie Morbus Hodgkin ; embryonale Tumoren wie z.B. Neuroblastom, Nephroblastom, Teratom, Adamantinom und Retroblastom, Haemangiom, Chordom, Odontom und Craniopharyngiom ; Hamartome wie z.B. Lymphoangiom, Exostosen, Neurofibromatose ; Melanome ; Lymphome ; Hepatoblastome ; Mammakarzinom ; Cervixkarzinom ; Choriokarzinom, Adrenoacanthom ; Leiomyom, Androblastom ; Arrhenoblastom ; Sertolizelltumor ; Granulosa- und Theca-Zell-Tumor ; Germinom und Seminom, Ovarial- und Vulvakarzinom ; Harnblasen- und Prostatakarzinom ; durch Schistosomiasis hervorgerufene Tumoren ; Astrocytom, Ependymogliome ; Glioblastome, Medulloblastom ; Oligodendrogliom, Spongioblastom ; Meningeom sowie Tumoren der Schwann'schen Scheidenzellen ; Pinealom ; Haemangioblastom, Osteoclastom, Ewings Tumor ; Multiples Myelom ; Mycosis fungoides ; Burkitt-Tumor ; Leukaemien wie z.B. akute und chronische lymphatische Leukaemie, akute und chronische Granulocytenleukaemie, akute und chronische Monocytenleukaemie sowie die Stammzell-Leukaemie ; Basaliom, Fibrom, Myom sowie vor allem die bei der chirurgischen Intervention einer lokalen Injektion zugänglichen Metastasen sämtlicher Tumorformen.

Ebenfalls beim Osteosarkom zeigen sich therapeutisch wertvolle Wirkungen der erfindungsgemäßen Verbindungen ; hier müssen diese jedoch mit einer Spezialvorrichtung unter Druck bis zu 600 atü injiziert werden. Darüber hinaus sind die erfindungsgemäßen Verbindungen anwendbar bei fibrosierenden Geweben jeglicher Art, insbesondere bei der Behandlung von Keloiden, Ulcera crura, Verbrennungsgeschwüren, Decubitalulcera sowie Clavi und Onychomykosen, Narbengewebe und zur Therapie und

Prophylaxe von Emboli und Thrombosen.

Ebenso können die erfindungsgemäßen Verbindungen eingesetzt werden zur Auflösung von Muttermalen, Atheromen und Lipomen sowie zur Beseitigung von tiefen, unter Umständen fistelnden Abszessen.

Zusätzlich können die erfindungsgemäßen Verbindungen eingesetzt werden zur Regeneration von Kavernomen und Tuberkulomen.

Die erfindungsgemäßen Verbindungen sind ebenfalls einsetzbar zur narbenfreien Regeneration der Substanzdefekte bei der Lepra und anderen Haut-, Schleimhaut- bzw. Epitheldefekten verschiedener Genese, vor allem solcher, die durch Infektionen mit Bakterien, Pilzen und Erregern von Tropenkrankheiten wie z.B. die der Leishmaniosen, Framboesia, Pinta u.a. bedingt sind.

## Beispiel 1

N-Acetyldehydrophenylalanyl-L-tryptophan

Eine Suspension von 18,7 g (0,1 Mol) L-Tryptophan in 40 ml Aceton wird unter Rühren mit 100 ml n Natronlauge versetzt und die entstandene Lösung anschließend mit einer zweiten Lösung von 20,4 g (0,1 Mol) 2-Methyl-4-benzyliden-5(4H)-oxazolon (R.M. Herbst, D. Shemin, Org. Synth. Coll. Vol. II, 1 (1943)) in 60 ml Aceton versetzt. Nach 2 1/2-stündigem Rühren bei Raumtemperatur wird die Reaktionslösung mit Aktivkohle behandelt, über Kieselgur filtriert und mit 100 ml n Salzsäure neutralisiert. Der ausgefallene Niederschlag wird abfiltriert, mit Wasser gewaschen und aus Ethanol/Wasser (1:1) umkristallisiert.

Ausbeute 27,8 g (71 % der Theorie). Fp. 215 °C.

$[\alpha]_D^{20} = -26{,}3$ °C (c = 0,5, Dimethylformamid)

Ebenso wurde hergestellt:

## Beispiel 2

N-Acetyldehydrophenylalanyl-D-tryptophan

wird analog Beispiel 1 erhalten aus 2-Methyl-4-benzyliden-5(4H)-oxazolon und D-Tryptophan.

Ausbeute 58,7 % der Theorie. Fp. 220 bis 221 °C.

$[\alpha]_D^{20} = +26{,}3$ °C (c = 0,5, Dimethylformamid)

## Beispiel 3

N-Acetyldehydrophenylalanyl-dehydrotryptophan

Zu einer Suspension von 3,9 g (10 mMol) N-Acetyldehydrophenylalanyl-L-tryptophan in 20 ml trockenem Tetrahydrofuran gibt man bei Raumtemperatur unter Rühren 2,1 g (10 mMol) Dicyclohexylcarbodiimid. Nach 2-stündigem Rühren und anschließendem Stehen über Nacht im Kühlschrank wird ausgefallener Dicyclohexylharnstoff abgesaugt und mit Tetrahydrofuran ausgewaschen. Das Filtrat wird eingedampft und das zurückbleibende gesättigte Rohoxazolon in 80 ml 1,2-Dimethoxyethan (zur Trocknung über neutrales Aluminiumoxid filtriert) gelöst. Unter Rühren werden nacheinander 2,3 g (10 mMol) 2,3-Dichlor-5,6-dicyan-p-benzochinon und 1,32 ml (10 mMol) 2,4,6-Trimethylpyridin zugegeben. Der Ansatz wird 6 Stunden bei Raumtemperatur aufbewahrt, eingedampft und der Rückstand aus Ethylacetat an Kieselgel chromatographiert. Die das ungesättigte Oxazolon enthaltenden Fraktionen werden eingedampft, mit Wasser ausgerührt und getrocknet.

Ausbeute 2,2 g (59,5 % der Theorie). Fp. 207 bis 209 °C.

11,5 g (30,9 mMol) des wie vorstehend hergestellten Oxazolons der Titelverbindung werden in 80 ml Aceton suspendiert und bei Raumtemperatur unter Rühren tropfenweise mit 30,9 ml 2 n Natronlauge versetzt. Die entstehende Lösung wird 1 Stunde bei Raumtemperatur aufbewahrt, dann mit 400 ml Wasser verdünnt und mit Dichlormethan extrahiert. Der nach dem Ansäuern der wäßrigen Phase mit Zitronensäure auskristallisierende Niederschlag wird abgesaugt, mit Wasser gewaschen und 2 mal mit Ethylacetat ausgerührt und bei 100 °C getrocknet.

Ausbeute 8 g (66,7 % der Theorie). Fp. 175 bis 178 °C.

**Ansprüche**

1. Dehydropeptide der allgemeinen Formel I

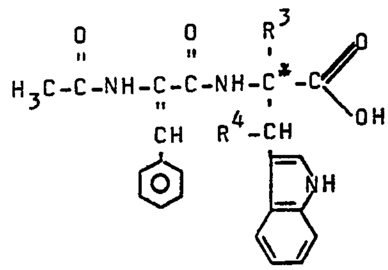

(I)

in welcher

$R^3$ und $R^4$ jeweils für ein Wasserstoffatom oder zusammen für eine zusätzliche Bindung zwischen den beiden C-Atomen stehen,
in ihrer racemischen und in ihrer optisch aktiven Form, sowie ihre physiologisch verträglichen Salze.

2. Verfahren zur Herstellung von Dehydropeptiden der allgemeinen Formel I

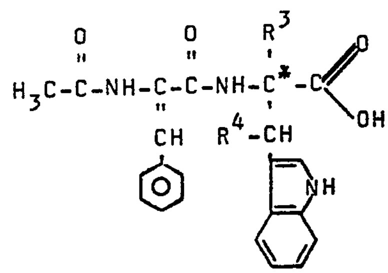

(I)

in welcher

$R^3$ und $R^4$ jeweils für ein Wasserstoffatom oder zusammen für eine zusätzliche Bindung zwischen den beiden C-Atomen stehen,
in ihrer racemischen und in ihrer optisch aktiven Form, sowie ihre physiologisch verträglichen Salze, dadurch gekennzeichnet, daß man

a) für Verbindungen, in denen $R^3$ und $R^4$ jeweils Wasserstoff bedeuten,
Oxazole der Formel II

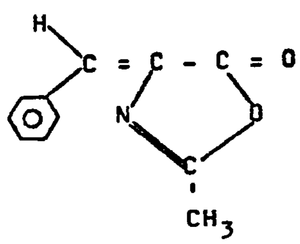

(II)

mit Tryptophanderivaten der Formel III

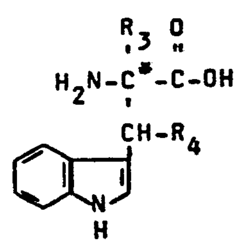

(III)

in welcher

$R^3$ und $R^4$ jeweils Wasserstoff bedeuten und

C* in der Racemat- oder in D- oder L-Form vorliegt,

in Gegenwart von organischen Lösungsmitteln, Wasser oder Gemischen aus beiden, bei Temperaturen zwischen 0 und 80 °C umsetzt oder

b) für Verbindungen der allgemeinen Formel I, in welcher $R^3$ und $R^4$ für eine zusätzliche Bindung zwischen den beiden C-Atomen stehen,
gesättigte Oxazole der Formel IV

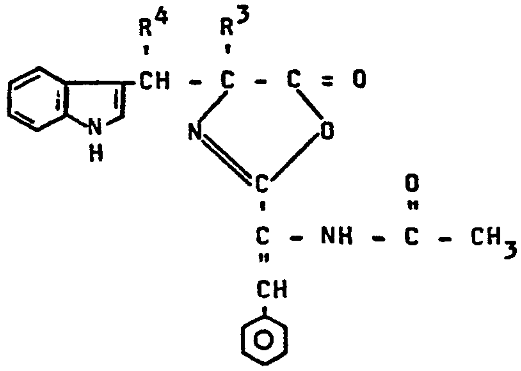

(IV)

in welcher

$R^3$ und $R^4$ für jeweils ein Wasserstoffatom stehen,

in Gegenwart von inerten organischen Lösungsmitteln und von tertiären Aminen oxidiert und anschließend in üblicher Weise in Gegenwart von Wasser enthaltenden Verdünnungsmitteln bei Temperaturen zwischen 0 und 70 °C zu Verbindungen der allgemeinen Formel I verseift.

3. Arzneimittel enthaltend mindestens 1 Dehydropeptid der allgemeinen Formel I gemäß Anspruch 1.

4. Verfahren zur Herstellung von Arzneimitteln gemäß Anspruch 3, dadurch gekennzeichnet, daß man Dehydropeptide der allgemeinen Formel I gemäß Anspruch 1 gegebenenfalls unter Verwendung von Hilfs- und Trägerstoffen in eine geeignete Applikationsform überführt.

5. Verwendung von Dehydropeptiden der allgemeinen Formel I gemäß Anspruch 1 bei der Herstellung von Arzneimitteln zur Bekämpfung von malignen Geweben und Tumoren.

**Claims**

1. Dehydropeptides of the general formula I

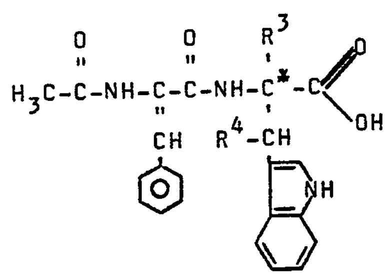

(I)

in which

$R^3$ and $R^4$ in each case represent a hydrogen atom or together represent an additional bond between the two C atoms,

in their racemic form and in their optically active form, and their physiologically acceptable salts.

2. Process for the preparation of dehydropeptides of the general formula I

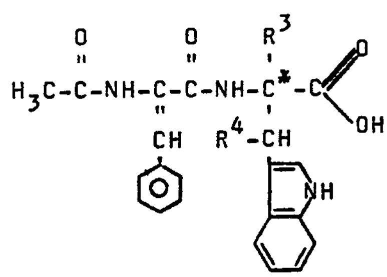

(I)

in which

$R^3$ and $R^4$ in each case represent a hydrogen atom or together represent an additional bond between the two C atoms,

in their racemic form and in their optically active form, and their physiologically acceptable salts, characterised in that

a) for compounds in which $R^3$ and $R^4$ in each case denote hydrogen, oxazoles of the formula II

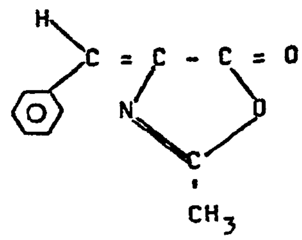

(II)

are reacted with tryptophan derivatives of the formula III

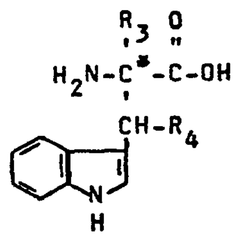

(III)

in which

$R^3$ and $R^4$ in each case denote hydrogen and

C* is in the racemate form or in the D-form or L-form,

in the presence of organic solvents, water or mixtures of both, at temperatures between 0 and 80 °C or

b) for compounds of the general formula I in which $R^3$ and $R^4$ represent an additional bond between the two C atoms, saturated oxazoles of the formula IV

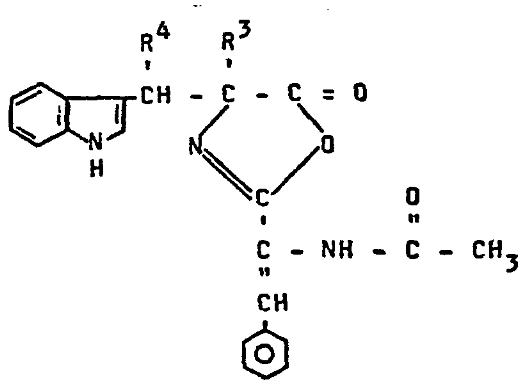

in which

$R^3$ and $R^4$ in each case represent a hydrogen atom, are oxidised in the presence of inert organic solvents and of tertiary amines and the products are then saponified in the customary manner in the presence of diluents containing water, at temperatures between 0 and 70 °C, to give compounds of the general formula I.

3. Medicaments containing at least 1 dehydropeptide of the general formula I according to Claim 1.

4. Process for the preparation of medicaments according to Claim 3, characterised in that dehydropeptides of the general formula I according to Claim 1 are converted into a suitable form of administration, using auxiliaries and excipients if appropriate.

5. Use of dehydropeptides of the general formula I according to Claim 1 in the preparation of medicaments for combating malignant tissues and tumours.

**Revendications**

1. Déhydropeptides de formule générale

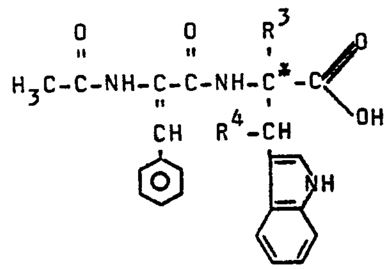

(I)

dans laquelle

$R^3$ et $R^4$ représentent chacun un atome d'hydrogène ou bien représentent ensemble une liaison supplémentaire entre les deux atomes de carbone,

sous leur forme racémique et sous leur forme optiquement active, ainsi que leurs sels physiologiquement acceptables.

2. Procédé pour la préparation des composés de formule générale I

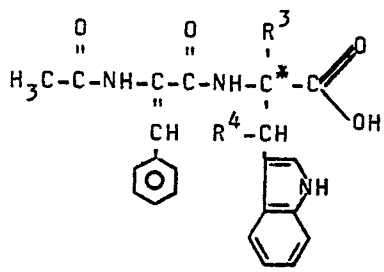

(I)

dans laquelle

$R^3$ et $R^4$ représentent chacun un atome d'hydrogène ou bien représentent ensemble une liaison supplémentaire entre les deux atomes de carbone,

sous leur forme racémique et sous leur forme optiquement active, ainsi que leurs sels physiologiquement acceptables, caractérisé en ce que

a) pour les composés dans lesquels $R^3$ et $R^4$ représentent chacun l'hydrogène, on fait réagir des oxazoles de formule II

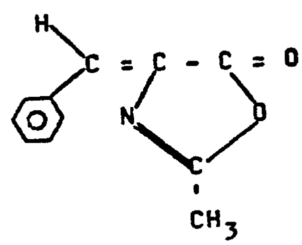

(II)

avec des dérivés de tryptophane de formule III

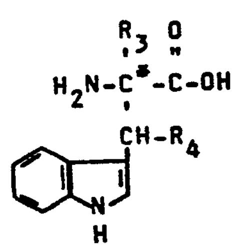

(III)

dans laquelle

$R^3$ et $R^4$ représentent chacun l'hydrogène et

C* est sous la forme de racémate ou sous la forme D ou L, à des températures comprises entre 0 et 80 °C, en présence de solvants organiques, d'eau ou de mélanges des deux, ou bien

b) pour les composés de formule générale I, dans laquelle $R^3$ et $R^4$ représentent une liaison supplémentaire entre les deux atomes de carbone, on oxyde des oxazoles saturés de formule IV

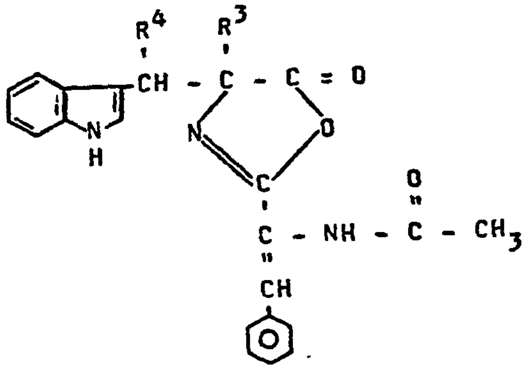

dans laquelle

$R^3$ et $R^4$ représentent chacun un atome d'hydrogène, en présence de solvants organiques inertes et d'amines tertiaires, et ensuite on saponifie de la manière habituelle en présence de solvant contenant de l'eau à des températures comprises entre 0 et 70 °C en composés de formule générale I.

3. Médicaments contenant au moins un déhydropeptide de formule générale I selon la revendication 1.

4. Procédé pour la préparation de médicaments selon la revendication 3, caractérisé en ce que l'on transforme des déhydropeptides de formule générale I, selon la revendication 1, en une forme d'application appropriée en utilisant des agents auxiliaires et des supports.

5. Utilisation de déhydropeptides de formule générale I, selon la revendication 1, dans la préparation de médicaments pour la lutte contre les tissus et tumeurs malins.